# NEUE EUROPÄISCHE PATENTSCHRIFT

(11) **EP 0 589 977 B2**
(45) Veröffentlichungstag und Bekanntmachung des Hinweises auf die Entscheidung über den Einspruch: **18.10.2000**
(45) Hinweis auf die Patenterteilung: 01.03.1995
(21) Anmeldenummer: 92912341.2
(22) Anmeldetag: 17.06.1992
(51) Int. Cl.: C07C 33/046, C07C 29/38

(54) **VERFAHREN UND VORRICHTUNG ZUR SYNTHESE VON BUTINDIOL**
PROCESS AND DEVICE FOR SYNTHESISING BUTYNEDIOL
PROCEDE ET DISPOSITIF POUR LA SYNTHESE DU BUTYNEDIOL

(30) Priorität: 20.06.1991 DE 4120446
(43) Veröffentlichungstag der Anmeldung: 06.04.1994
(73) Patentinhaber: Linde Aktiengesellschaft, 65189 Wiesbaden (DE); YUKONG LIMITED, Yongdungpo-gu, Seoul (KR)
(72) Erfinder: WIESSNER, Frank, D-8000 München 60 (DE)
(74) Vertreter: Kasseckert, Rainer
(86) Internationale Anmeldenummer: EP9201376
(87) Internationale Veröffentlichungsnummer: WO9300318

(56) Entgegenhaltungen:
- DE-A- 2 206 693
- DE-A- 2 514 990
- DE-A- 2 814 447
- DE-A- 3 228 783
- US-A- 3 154 589
- US-A- 4 117 248

## Beschreibung

Die Erfindung betrifft ein Verfahren zur Synthese von Butindiol aus einer zumindest Formaldehyd enthaltenden wäßrigen Lösung durch Reaktion mit Acetylen in Gegenwart eines suspensierten Katalysators, wobei die Lösung in einer Kaskade durch mehrere Reaktoren geführt wird, indem die aus dem ersten bis vorletzten Reaktor der Kaskade abgezogene Lösung in den in der Kaskade nächstfolgenden Reaktor eingespeist wird, wobei in die Reaktoren Acetylen eingeleitet wird, wobei aus dem in der Kaskade zuletzt durchströmten Reaktor eine Butindiol-reiche Lösung abgezogen wird und wobei durch Abtrennung des Katalysators aus der Lösung ein Butindiol-haltiger Produktstrom gewonnen wird.

Aus der DE-PS 28 14 447 ist ein Niederdruck-Ethinylierungsverfahren zur Herstellung von Butindiol aus Formaldehyd und Acetylen unter Anwendung eines suspensierten, fein verteilten Katalysators bekannt. Formaldehyd und Acetylen werden, unterstützt durch den suspensierten Katalysator, in einer Kaskade von Reaktoren in Butindiol umgewandelt. Rest-Formaldehyd, gelöstes Acetylen, bereits hergestelltes Butindiol, Wasser und der Suspensionskatalysator werden durch Reaktoren und Leitungen bis in eine Abtrennvorrichtung geführt, wo ein Katalysator-haltiger Strom von der Produktlösung abgetrennt und anschließend erneut in die Reaktorkaskade rückgeführt wird. Die Abtrennung des Katalysator-haltigen Stromes von der Produktlösung erfolgt im bekannten Verfahren nach dem obengenannten Patent über einen Druckblattfilter.

Es bestehen bei der beschriebenen Verfahrensführung, auch bei Benutzung anderer Abtrennvorrichtungen, folgende Nachteile:
Die bislang bekannten Katalysatoren werden unter Acetylenmangel desaktiviert. Diesem Sachverhalt wird üblicherweise dadurch Rechnung getragen, daß im Reaktionsgemisch ein Acetylenüberschuß eingestellt wird. Dennoch bleibt die Gefahr einer Schädigung des Katalysators, vor allem in den Leitungen und der Abtrennvorrichtung, bestehen, da der Katalysator sich absetzt und damit eine punktuelle Katalysatorverdichtung auftritt.

Der entscheidende Nachteil des bekannten Verfahrens ist dadurch bedingt, daß immer ein erheblicher Teil des erzeugten Butindiols in der Katalysator-haltigen Lösung aus der Abtrennvorrichtung zur Reaktorkaskade rückgeführt wird, da der Suspensionskatalysator fließfähig gehalten werden muß. Das rückgeführte Butindiol wirkt aber hemmend auf die Reaktion.

Damit verringert sich die im Verfahren insgesamt erzielte Umwandlungsrate der Ausgangskomponenten in Butindiol. Außerdem steigt der Gehalt an Rest-Formaldehyd in der Produktlösung. Dieser erhöhte Rest-Formaldehydgehalt bringt aber bei einer Weiterverarbeitung des Produktstromes, beispielsweise bei einer gewünschten Hydrierung des Butindiols aus dem Produktstrom, zusätzliche Probleme mit sich.

Die Gefahr der Katalysatorschädigung durch Acetylenmangel nimmt mit sinkender Temperatur ab. Der Katalysator kann also bei verringerter Temperatur aufkonzentriert werden. Allerdings verringert sich die Fließfähigkeit der Lösung ebenfalls mit sinkender Temperatur, wodurch das Filtrierverhalten der Lösung verschlechtert wird. So geht beispielsweise der Filtratstrom bei einer Senkung der Temperatur von etwa 80°C auf etwa 20°C bei ansonsten gleichen Filterbedingungen auf etwa ein Viertel des ursprünglichen Filtratstromes zurück. Bei einer Verfahrensführung mit tiefen Temperaturen wird dadurch der apparative Aufwand erhöht.

Der Erfindung liegt daher die Aufgabe zugrunde ein Verfahren der eingangs genannten Art aufzuzeigen, welches die aufgezeigten Nachteile auf einfache Art und Weise vermeidet.

Diese Aufgabe wird erfindungsgemäß dadurch gelöst, daß die Abtrennung des Katalysators von der Lösung pro einzelnem Reaktor der Kaskade erfolgt. und der abgetrennte Katalysator in dem jeweiligen Reaktor verbleibt bzw. ummmittelbar in den jeweiligen Reaktor zurüchgeführt wird.

Erfindungsgemäß wird der Katalysator in den einzelnen Stufen der Reaktorkaskade zurückgehalten. Dabei kann erfindungsgemäß die Abtrennung des Katalysators von der Lösung außerhalb der Reaktoren durchgeführt werden. Der Katalysator wird, nachdem er mit der Lösung aus dem einzelnen Reaktor abgezogen wurde, von dieser abgetrennt und wieder demselben Reaktor zugeführt. Die Katalysator-freie Lösung wird in den nächstfolgenden Reaktor der Kaskade eingeleitet bzw. als Produktstrom vom letzten Reaktor der Kaskade abgezogen. Durch die Rückführung des Katalysators in den Reaktor wird zwar auch ein Teil der aus diesem Reaktor abgezogenen Lösung in diesen Reaktor rückgeführt, aber diese Lösung weist diesselbe Butindiol-Konzentration auf, wie sie im Reaktor vorliegt, so daß durch die Rückführung keine die Umsetzungsreaktion hemmende Butindiol-Anreicherung in den einzelnen Reaktoren auftritt wie beim Verfahren nach dem Stand der Technik.

Die Verweilzeit des Katalysators außerhalb der Reaktoren ist verglichen mit der nach Verfahren nach dem Stand der Technik kurz. In den Reaktoren läßt sich ein Acetylenüberschuß leicht einhalten. Eine Schädigung des Katalysators durch Acetylenmangel tritt daher im erfindungsgemäßen Verfahren nicht auf.

Mit besonderem Vorteil kann der Katalysator von der Lösung erfindungsgemäß aber auch innerhalb des Reaktors abgetrennt werden. Durch den Acetylenüberschuß in den Reaktoren bleibt eine Katalysator- schädigung ausgeschlossen. Eine Rückführung von Lösung in die Reaktoren tritt in diesem Fall nicht auf.

Als Reaktoren können im erfindungsgemäßen Verfahren alle Reaktoren verwendet werden, die eine gute Verteilung der Reaktionskomponenten und des Katalysators im Reaktor ermöglichen. Insbesondere eignen sich hierzu Rührkessel. Die Abtrennung des Katalysators von der Lösung und damit das Abziehen einer Katalysator-freien Lösung von den Stufen der Reaktorkaskade kann durch Filter aber auch durch andere Abtrennvorrichtungen, wie beispielsweise Hydrozyklone als Abtrennvorrichtung außerhalb der Reaktoren, erreicht werden. Die Abtrennung des Katalysators von der Lösung erfolgt im erfindungsgemäßen Verfahren bei Reaktionstemperatur. Diese liegt zwischen etwa 60 und 100°C, vorzugsweise zwischen 80 und 90°C. Bei diesen hohen Temperaturen ist die Suspension dünnflüssig, was die Abtrennung wesentlich erleichtert. Die Reaktion erfolgt bei Umgebungsdruck bzw. bei leicht erhöhtem Druck.

Vorteilhaft für die Umwandlung der Ausgangskomponenten in Butindiol wirkt sich aus, wenn in den Reaktoren eine Katalysatormenge von 50 bis 200g, vorzugsweise von 75 bis 125g, pro Liter Lösung vorliegt.

In Ausgestaltung des erfindungsgemäßen Verfahrens wird eine Teilmenge des in den Reaktoren der Kaskade befindlichen suspensierten Katalysators aus den Reaktoren zusätzlich zur katalysatorfreien Lösung abgezogen und durch eine entsprechende Menge an Katalysator ersetzt. Damit kann sichergestellt werden, daß nicht die normale Alterung des Katalysators gerade im kontinuierlichen Betrieb zu einer sinkenden Umwandlungsrate in Butindiol führt.

Der Austausch des Katalysators kann diskontinuierlich erfolgen. Mit Vorteil wird jedoch der Katalysator kontinuierlich aus den Reaktoren abgezogen und diesen zugeführt.

In Weiterbildung des erfindungsgemäßen Verfahrens kann der aus den Reaktoren abgezogenen Katalysator zumindest teilweise einer Regeneration zugeführt werden. Denn damit entsteht der Vorteil, daß zumindest ein Teil des abgezogenen Katalysators wieder nutzbringend im Verfahren eingesetzt werden kann.

Mit Vorteil wird regenerierter und/oder frischer suspendierter Katalysator in die Reaktoren eingeleitet. Bei dieser erfindungsgemäßen Verfahrensführung läßt sich eine besonders hohe Umwandlung der Ausgangskomponeneten in Butindiol erreichen. Der suspendierte Katalysator kann dabei direkt oder mit der Lösung in die Reaktoren geleitet werden. Im letztgenannten Fall wird bereits außerhalb des Reaktors der Katalysator mit der Lösung vermischt.

Als Katalysatoren im erfindungsgemäßen Verfahren eignen sich alle für die Butindiolproduktion bekannten Suspensionskatalysatoren. Besonders gute Ergebnisse wurden mit Katalysatoren aus einer Kupferverbindung auf einem Aluminiumoxidträger erzielt.

Die Erfindung betrifft ferner eine Vorrichtung zur Durchführung des eingangs beschriebenen Verfahrens mit mehreren Reaktoren, die über Verbindungsleitungen in einer Kaskade verbunden sind und jeweils Zuleitungen für die Lösung und für Acetylen, sowie Leitungen zum Abzug für die Lösung und die in den Reaktoren freiwerdenden Gase aufweisen. Erfindungsgemäß ist in jedem Reaktor eine Trenneinrichtung zur Abtrennung einer katalysatorfreien Lösung angeordnet.

Diese Trenneinrichtung kann aus einem Filter mit Filterkerzen oder Filterflächen bestehen. Es sind aber auch Trenneinrichtungen wie beispielsweise Zyklone außerhalb des Reaktors einsetzbar. Im Falle einer Trenneinrichtung außerhalb des Reaktors führt eine Leitung vom Reaktor zur Trenneinrichtung, über die Katalysator und Lösung in die Trenneinreichtung geleitet werden, eine Leitung von der Trenneinrichtung zum Reaktor, über die der Katalysator in den Reaktor rückgeführt wird, sowie eine Leitung aus der Trenneinrichtung, über die Katalysator-freie Lösung aus der Stufe der Reaktorkaskade abgezogen wird.

In einer speziellen Ausführungsform der erfindungsgemäßen Vorrichtung sind die Reaktoren oder die Zuleitungen für die Lösung zusätzlich mit weiteren Leitungen ausgestattet, über die Katalysator eingespeist wird. Außerdem führt aus jedem Reaktor eine Leitung, über die Katalysator abgezogen wird.

Vorteilhafterweise sind die in einer Weiterbildung der Erfindung die Verbindungsleitungen mit Pumpen ausgestattet. Da über die Verbindungsleitungen lediglich katalysatorfreie Lösung von Reaktor zu Reaktor geleitet wird, können in den Verbindungsleitungen problemlos einfache Pumpen eingesetzt werden, da die Gefahr einer Katalysatorschädigung in den Pumpen nicht besteht.

Die Erfindung wird im folgenden anhand eines Ausführungsbeispiels näher erläutert.

Hierbei zeigt:

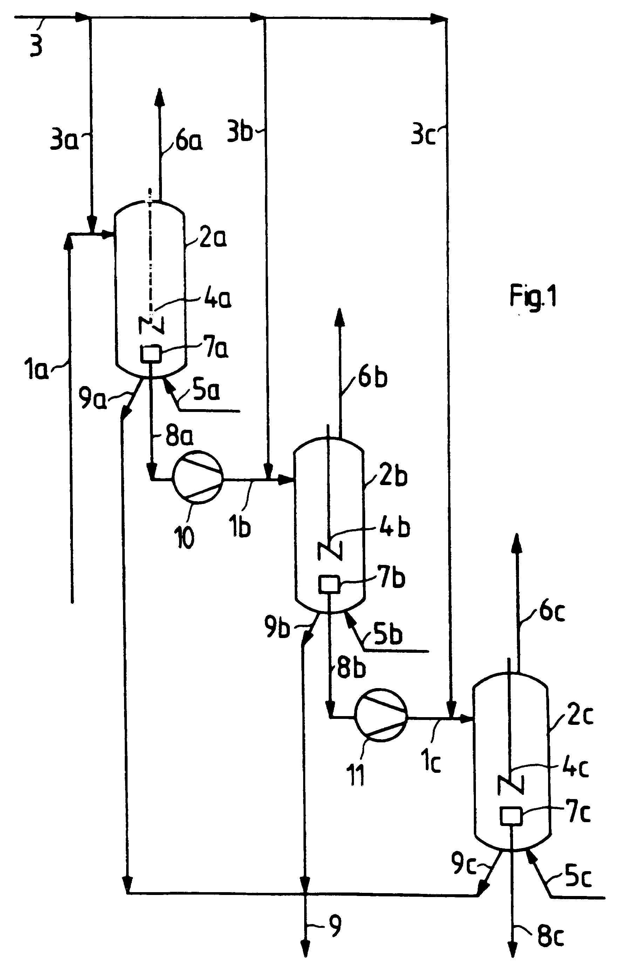

Figur 1 eine erfindungsgemäße Reaktorkaskade.

In Figur 1 ist ein Schema einer erfindungsgemäßen Reaktorkaskade mit drei Rührkesseln dargestellt. Über Leitung 1a wird zumindest Formaldehyd-haltige Lösung in den ersten Rührkessel 2a geleitet. Suspensierter Katalysator wird über Leitung 3 und Leitung 3a ebenfalls in Rührkessel 2a eingeführt. Der Rührkessel 2a ist mit dem Rührer 4a ausgestattet. Der Rührer 4a gewährleistet eine gute Durchmischung der Reaktionslösung. Über Leitung 5a wird Acetylen in den Reaktorraum eingegast. Im Rührkessel findet eine teilweise Umwandlung des Formaldehyds und Acetylens in Butindiol statt. Überschüssige Gase, im wesentlichen Acetylen, werden über Leitung 6a aus dem Rührkessel 2a abgezogen. Im unteren Bereich des Rührkessels 2a ist ein Filter 7a angeordnet, der den suspensierten Katalysator im Rührkessel 2a zurückhält. Der Filter 7a ist im Rührkessel 2a so angeordnet, daß aufgrund der Strömungsbedingungen im Rührkessel 2a ein Zusetzen des Filters 7a vermieden wird. Durch den Filter 7a wird eine bereits Butindiol-haltige Reaktionslösung über Leitung 8a aus dem Rührkessel 2a abgezogen. Das Reaktionsgemisch wird über Pumpe 10 der zweiten Stufe der Reaktorkaskade zugeführt. Vom Boden des Rührkessels 2a wird über Leitung 9a suspensierter Katalysator abgezogen.

Über Leitung 1b wird das Reaktionsgemisch aus der ersten Stufe der Kaskade in die zweite Stufe geleitet. Den Kern der zweiten Stufe der Kaskade bildet der Rührkessel 2b. Rührkessel 2b wird entsprechend Rührkessel 2a betrieben und weist daher äquivalente Einbauten, Zu- und Ableitungen auf. In Figur 1 ist noch eine dritte Stufe der Kaskade mit dem Rührkessel 2c mit ebenfalls äquivalenten Einbauten, Zu- und Ableitungen dargestellt. Die Äquivalenz ist in Figur 1 durch identische Bezugsziffern verdeutlicht, die mit dem Buchstaben a, b, bzw. c zur Kennzeichnung der ersten, zweiten bzw. dritten Stufe der Reaktorkaskade versehen sind. Die aus der zweiten Stufe der Kaskade über Leitung 8b abgezogene Lösung wird über die Pumpe 11 und Leitung 1c in die dritte Stufe der gezeigten Reaktorkaskade geleitet. In jeder Stufe der Kaskade findet eine weitere Umwandlung in Butindiol statt, die Zahl der verwendeten Kaskadenstufen ist durch die Erfindung nicht vorgegeben, sondern sie hängt von spezifischen Randbedingungen einer Anlage ab.

Aus der letzten Stufe der Reaktorkaskade wird ein katalysatorfreier, Butindiol-reicher Produktstrom gewonnen, der beliebig weiterverwendet oder aufbereitet werden kann. Im Ausführungsbeispiel wird über Leitung 8c ein Butindiol-reicher Produktstrom aus dem Rührkessel 2c abgezogen.

Die aus den Rührkesseln 2a, 2b bzw. 2c geleiteten Katalysatorströme 9a, 9b bzw 9c können über Leitung 9 einer in Figur 1 nicht dargestellten Regenerierung zugeführt werden. Die bei der Regenerierung gewonnene Katalysatorsuspension kann über Leitung 3 nutzbringend in das Verfahren rückgeführt werden.

Insgesamt lassen sich die Vorteile des erfindungsgemäßen Verfahrens bzw. der erfindungsgemäßen Vorrichtung folgendermaßen zusammenfassen: Die Reaktoren, speziell die Rührkessel, können bei gleichem Butindiol-Umsatz wesentlich kleiner gebaut werden. Damit fallen auch Rührer und Begasungssystem deutlich kleiner aus. Die Abtrennung der Katalysatorsuspension von der Lösung erfolgt über eine einfache Abtrenneinrichtung, beispielsweise über einfache, übliche Filter. Die Filterbedingungen sind aufgrund der hohen Temperaturen und der Strömungsbedingungen besonders günstig. Das erfindungsgemäße Verfahren bzw. die erfindungsgemäße Vorrichtung verhindert irreversible Schäden am Suspensionskatalysator. Die Ausfallsicherheit einer Anlage zur Butindiolproduktion wird durch die Erfindung wesentlich gesteigert.

## Patentansprüche

1. Verfahren zur Synthese von Butindiol aus einer zumindest Formaldehyd enthaltenden wäßrigen Lösung durch Reaktion mit Acetylen in Gegenwart eines suspensierten Katalysators, wobei die Lösung in einer Kaskade durch mehrere Reaktoren geführt wird, indem die aus dem ersten bis vorletzten Reaktor der Kaskade abgezogene Lösung in den in der Kaskade nächstfolgenden Reaktor eingespeist wird,
wobei in die Reaktoren Acetylen eingeleitet wird,
wobei aus dem in der Kaskade zuletzt durchströmten Reaktor eine Butindiol-reiche Lösung abgezogen wird und
wobei durch Abtrennung des Katalysators aus der Lösung ein Butindiol-haltiger Produktstrom gewonnnen wird,
**dadurch gekennzeichnet,**
daß die Abtrennung des Katalysators von der Lösung pro einzelnem Reaktor der Kaskade erfolgt. und der abgetrennte Katalysator in dem jeweiligen Reaktor verbleibt bzw. unmittelbar in den jeweiligen Reaktor zurückgeführt wird.

2. Verfahren nach Anspruch 1, dadurch gekennzeichnet, daß in den Reaktoren eine Katalysatormenge von 50 bis 200g, vorzugsweise von 75 bis 125g, pro Liter Lösung enthalten ist.

3. Verfahren nach einem der Ansprüche 1 oder 2, dadurch gekennzeichnet, daß eine Teilmenge des in den Reaktoren der Kaskade befindlichen Katalysators aus dem Reaktor abgezogen und eine entsprechende Menge an Katalysator den Reaktoren zugeführt wird.

4. Verfahren nach Anspruch 3, dadurch gekennzeichnet, daß kontinuierlich Katalysator abgezogen und zugeführt wird.

5. Verfahren nach einem der Ansprüche 3 oder 4, dadurch gekennzeichnet, daß der aus dem Reaktor abgezogene Katalysator zumindest teilweise einer Regenerierung zugeführt wird.

6. Verfahren nach den Ansprüchen 3 bis 5, dadurch gekennzeichnet, daß als in die Reaktoren eingeleiteter Katalysator regenerierter und/oder frischer Katalysator eingesetzt wird.

7. Verfahren nach einem der Ansprüche 1 bis 6, dadurch gekennzeichnet, daß der Katalysator aus einer Kupferverbindung auf einem Aluminiumoxidträger besteht.

8. Vorrichtung zur Durchführung des Verfahrens nach einem der Ansprüche 1 bis 7 mit mehreren Reaktoren (2a, 2b, 2c), die über Verbindungsleitungen (8a/1b, 8b/1c) in einer Kaskade verbunden sind und jeweils Zuleitungen für die Lösung (1a, 1b, 1c) und für Acetylen (5a, 5b, 5c) sowie Leitungen zum einen Abzug für die Lösung (8a, 8b, 8c) und die in den Reaktoren freiwerdenden Gase (6a, 6b, 6c) aufweisen, **dadurch gekennzeichnet,** daß in jedem Reaktor (2a, 2b, 2c) eine Trenneinrichtung (7a, 7b, 7c) zur Abtrennung einer katalysatorfreien Lösung angeordnet ist.

9. Vorrichtung nach Anspruch 8, dadurch gekennzeichnet, daß die Reaktoren (2a, 2b, 2c) zusätzlich Leitungen (9a, 9b, 9c) besitzen, über die Katalysator abgezogen wird und daß in die Reaktoren (2a, 2b, 2c) oder in die Zuleitungen der Lösung in den Reaktoren (1a, 1b, 1c) Leitungen zum Einleiten des suspensierten Katalysators (3a, 3b, 3c) führen.

10. Vorrichtung nach einem der Ansprüche 8 oder 9, dadurch gekennzeichnet, daß die Verbindungsleitungen (8a/1b, 8b/1c,) mit Pumpen (10, 11) ausgestattet sind.

## Claims

1. Process for synthesis of butynediol from an aqueous solution at least containing formaldehyde through reaction with acetylene in the presence of a suspended catalyst, in which the solution is passed in a cascade though a plurality of reactors, the solution removed from the first to the penultimate reactor in the cascade being fed into the next reactor in the cascade, acetylene is introduced into the reactors, a solution rich in butynediol is removed from the last reactor passed through in the cascade and a stream of product containing butynediol is obtained by separating the catalyst from the solution, **characterised** in that the separation of the catalyst from the solution is effected for each individual reactor in the cascade and the separated catalyst remains in each individual reactor and is fed back into each individual reactor respectively.

2. Process according to claim 1, characterised in that the reactors contain 50 to 200g, preferably 75 to 125g, of catalyst per litre of solution.

3. Process according to one of claims 1 or 2, characterised in that a part of the quantity of catalyst in the reactors in the cascade is removed from the reactor and a corresponding quantity of catalyst is fed into the reactors.

4. Process according to claim 3, characterised in that catalyst is removed and fed in continuously.

5. Process according to one of claims 3 or 4, characterised in that the catalyst removed from the reactor is sent at least in part for regeneration.

6. Process according to claims 3 to 5, characterised in that regenerated and/or fresh catalyst is used as the catalyst introduced into the reactors.

7. Process according to one of claims 1 to 6, characterised in that the catalyst consists of a copper compound on an aluminium oxide carrier.

8. Device for implementing the process according to one of claims 1 to 7 with a plurality of reactors (2a, 2b, 2c) which are connected by means of connecting lines (8a/1b, 8b/1c) in a cascade and each exhibit feed lines for the solution (1a, 1b, 1c) and for acetylene (5a, 5b, 5c) and lines for removal of the solution (8a, 8b, 8c) and the gases released in the reactors (6a, 6b, 6c), **characterised** in that a separating apparatus (7a, 7b, 7c) is disposed in each reactor (2a, 2b, 2c) to sepreate a solution free of catalyst.

9. Device according to claim 8, characterised in that the reactors (2a, 2b, 2c) additionally have lines (9a, 9b, 9c) through which catalyst is removed, and in that lines for introduction of the suspended catalyst (3a, 3b, 3c) lead into the reactors (2a, 2b, 2c) or into the lines for feeding solution in to the reactors (1a, 1b, 1c).

10. Device according to one of claims 8 or 9, characterised in that the connecting lines (8a/1b, 8b/1c) are equipped with pumps (10, 11).

## Revendications

1. Procédé de synthèse de butynediol à partir d'une solution aqueuse contenant au moins du formaldéhyde, par réaction d'acétylène en présence d'un catalyseur en suspension, dans lequel la solution est passée à travers plusieurs réacteurs en cascade en introduisant la solution soutirée du premier jusqu' à l'avant-dernier réacteur de la cascade, dans le réacteur suivant dans la cascade, dans lequel on introduit de l'acétylène dans les réacteurs, dans lequel on soutire du réacteur traversé en dernier dans la cascade une solution riche en butynediol, et dans lequel par séparation du catalyseur de la solution, on récupère un courant de produit contenant du butynediol, **caractérisé** en ce que la séparation du catalyseur de la solution est effectuée pur chaque réacteur individuel de la cascade et le catalyseur séparé reste dans le réacteur respectif ou est renvoyé immédiatement dans le réacteur respectif.

2. Procédé selon la revendication 1, caractérisé en ce que, dans les réacteurs est employée une quantité de catalyseur comprise entre 50 et 200 g, de préférence entre 75 et 125 g, par litre de solution.

3. Procédé selon l'une des revendications 1 ou 2, caractérisé en ce que, on soutire des réacteurs une quantité partielle du catalyseur se trouvant dans les réacteurs de la cascade et en ce qu'on introduit dans les réacteurs une quantité correspondante de catalyseur.

4. Procédé selon la revendication 3, caractérisé en ce que, du catalyseur est soutiré et ajouté en continu.

5. Procédé selon l'une des revendications 3 ou 4, caractérisé en ce que, le catalyseur soutiré du réacteur est soumis au moins partiellement à une régénération.

6. Procédé selon les revendications 3 à 6, caractérisé en ce que, on introduit dans les réacteurs comme catalyseur du catalyseur régénéré et/ou frais.

7. Procédé selon l'une des revendications 1 à 6, caractérisé en ce que, le catalyseur consiste on un composé de cuivre fixé sur un support à base d'oxyde d'aluminium.

8. Dispositif pur mettre e oeuvre le procédé selon l'une des revendications 1 à 7 avec plusieurs réacteurs (2a, 2b, 2c), reliés en cascade via des conduites de raccordement (8a/1b, 8b/1c) et qui comportent chacun des conduits d'amenée de la solution (1a, 1b, 1c) et de l'acétylène (5a, 5b, 5c) ainsi que des conduits de soutirage de la solution (8a, 8b, 8c) et des gaz qui ont été libérés dans les réacteurs (6a, 6b, 6c), ce dispositif étant **caractérisé** en ce que, dans de chaque réacteur (2a, 2b, 2c) est disposé un organe de séparation (7a, 7b, 7c) permettant l'obtention d'une solution exempte de catalyseur.

9. Dispositif selon la revendication 8, caractérisé en ce que, les réacteurs (2a, 2b, 2c) comportent des conduites supplémentaires (9a, 9b, 9c), via lesquelles est soutiré le catalyseur et en ce que dans les réacteurs (2a, 2b, 2c) et les conduites d'amenée de la solution (1a, 1b, 1c) dans les réacteurs débouchent des conduites pour l'amenée de catalyseur en suspension (3a, 3b, 3c).

10. Dispositif selon l'une des revendications 8 ou 9, caractérisé en ce que, les conduites de liaison (8a,/1b, 8b/1c) sont équipées de pompes (10, 11).
